# FASCICULE DE BREVET EUROPEEN

(11) **EP 2 207 480 B1**
(45) Date de publication et mention de la délivrance du brevet: **20.04.2011**
(21) Numéro de dépôt: 08839091.9
(22) Date de dépôt: 23.09.2008
(51) Int. Cl.: A61B 5/113, A61B 5/0488, A61B 5/08, A61M 16/00

(54) **DISPOSITIF DE DIAGNOSTIC RESPIRATOIRE**
ATEMDIAGNOSEVORRICHTUNG
RESPIRATORY DIAGNOSTIC DEVICE

(30) Priorité: 25.09.2007 FR 0757834
(43) Date de publication de la demande: 21.07.2010
(73) Titulaire: Université Joseph Fourier, 38041 Grenoble Cedex 9 (FR); Assistance Publique Hôpitaux De Paris, 75184 Paris Cedex 04 (FR)
(72) Inventeur: GUMERY, Pierre-Yves, 38000 Grenoble (FR); HEYER, Laurent, F-75010 Paris (FR)
(74) Mandataire: de Beaumont, Michel
(86) Numéro de dépôt international: PCT/FR2008/051699
(87) Numéro de publication internationale: WO 2009/050364

(56) Documents cités:
- WO-A-99/62580
- US-A- 5 513 631
- PM SURATT, R MCTIER, SC WILHOIT: "Alae nasi electromyographic activity and timing in obstructive sleep apnea" J APPL PHYSIOL, 1985, pages 1252-1256, XP009099989
- A. VILA, P. LEVY, F. BLANC-JOUVAN, P.Y. GUMERY, G. QUEZEL, P.D. THUAN: "Respiratory muscle fatigue diagnosis and automatic analysis of EMG signals" ELECTROENCEPAHLOGRAPHY AND CLINICAL NEUTRONPHYSIOLOGY, 1988, pages 55-55, XP002479550
- L. HEYER, P.F. BACONNIER, A. EBERHARD, L. BIOT, J.P. VIALE, J.P. PERDRIX, P.Y. CARRY: "Non-invasive detection of respiratory muscles activity during assited ventilation" COMPTES RENDUES BIOLOGIES, 2002, pages 383-391, XP002479551
- AMERICAN THORACIC SOCIETY (ATS) AND THE EUROPEAN RESPIRATORY SOCIETY (ERS): "ATS/ERS Statement on Respiratory Muscle Testing" AM J RESPIR CRIT CARE MED, 2002, pages 518-624, XP002479552

## Description

### Domaine de l'invention

La présente invention concerne un dispositif d'analyse de l'état respiratoire d'un patient et notamment l'application d'un tel dispositif au contrôle d'un ventilateur respiratoire.

### Exposé de l'art antérieur

Dans divers cas cliniques, par exemple pour un patient en coma, le patient doit être mis sous assistance respiratoire. Il est alors relié à un appareil appelé respirateur ou ventilateur respiratoire.

Un dispositif d'analyse de l'état respiratoire d'un patient par des moyens qui permettent la détermination du retard rostro-caudal est décrite dans la demande de brevet US 5 513 631 A.

L'un des problèmes que pose l'assistance respiratoire est de déterminer le moment où on peut débrancher le patient pour que la respiration naturelle prenne le relais de l'assistance. Pour l'instant, les ventilateurs respiratoires ne sont pas équipés de dispositifs détecteurs permettant de déterminer quand il est possible d'effectuer une séparation dans des conditions de sécurité, et cette détermination est effectuée de façon empirique éventuellement avec l'assistance de systèmes experts électroniques.

### Résumé de l'invention

Ainsi, un objet particulier de la présente invention est de prévoir un dispositif de contrôle d'un ventilateur respiratoire permettant d'indiquer quand ce ventilateur peut être débranché sans risques.

La présente invention vise plus généralement un dispositif d'analyse de l'état respiratoire d'un patient, permettant par exemple de détecter des troubles respiratoires liés au sommeil (apnées obstructives et centrales du sommeil, syndrome d'augmentation des résistances des voies aériennes supérieures), de contrôler l'administration de drogues en cours d'anesthésie, et plus généralement de détecter des insuffisances respiratoires.

Pour atteindre ces objets, la présente invention prévoit un dispositif d'analyse de l'état respiratoire d'un patient comprenant des moyens de détermination de l'activité électromyographique au niveau des ailes du nez d'un patient et du début des contractions nasales ; des moyens de détermination de l'activité électromyographique au niveau du thorax du patient et du début des contractions inspiratoires thoraciques ; des moyens de détermination du retard rostro-caudal entre les débuts des contractions nasales et des contractions inspiratoires thoraciques ; des moyens de comparaison dudit retard à un seuil ; et des moyens de fourniture d'un signal d'alarme quand ledit retard est supérieur audit seuil.

La présente invention prévoit aussi un dispositif de contrôle d'un ventilateur respiratoire comprenant des moyens de détermination de l'activité électromyographique au niveau des ailes du nez d'un patient et du début des contractions nasales ; des moyens de détermination de l'activité électromyographique au niveau du thorax du patient et du début des contractions inspiratoires thoraciques ; des moyens de détermination du retard rostro-caudal entre les débuts des contractions nasales et des contractions inspiratoires thoraciques ; des moyens de comparaison dudit retard à un seuil ; et des moyens de fourniture d'un signal autorisant l'arrêt du ventilateur quand ledit retard est inférieur audit seuil.

Selon un mode de réalisation de la présente invention, les moyens de détermination d'activité électromyographique comprennent des électrodes cutanées.

Selon un mode de réalisation de la présente invention, un convertisseur analogique-numérique reçoit les signaux fournis par lesdites électrodes après un prétraitement, les signaux numériques étant soumis à divers moyens de filtrage et de mise en forme avant les opérations de comparaison.

Selon un mode de réalisation de la présente invention, le filtrage numérique comprend un filtrage adaptatif de signal cardiaque utilisant un signal d'ECG capté par d'autres électrodes cutanées et un filtrage à la fréquence du secteur.

Selon un mode de réalisation de la présente invention, le prétraitement est réalisé par des moyens d'amplification et de filtrage.

Selon un mode de réalisation de la présente invention, il est prévu des moyens de détermination de la moyenne glissante du retard rostro-caudal sur plusieurs phases respiratoires.

Selon un mode de réalisation de la présente invention, ledit seuil est réglable entre 200 et 1000 ms.

### Brève description des dessins

Ces objets, caractéristiques et avantages, ainsi que d'autres seront exposés en détail dans la description suivante de modes de réalisation particuliers faite à titre non-limitatif en relation avec les figures jointes parmi lesquelles :
la figure 1 illustre schématiquement le positionnement de détecteurs d'électromyogrammes selon un mode de réalisation de la présente invention ;
la figure 2 représente des électromyogrammes relevés respectivement au niveau des ailes du nez d'un patient et du thorax de celui-ci ; et
la figure 3 représente un exemple de dispositif de traitement de signaux d'électromyogrammes selon un mode de réalisation de la présente invention.

### Description détaillée

La présente invention prévoit un dispositif d'analyse comparative des électromyogrammes d'un patient relevés au niveau des ailes du nez de celui-ci et au niveau du thorax et de traitement du signal de comparaison pour fournir des signaux d'alerte, de plage de sécurité ou de diagnostic.

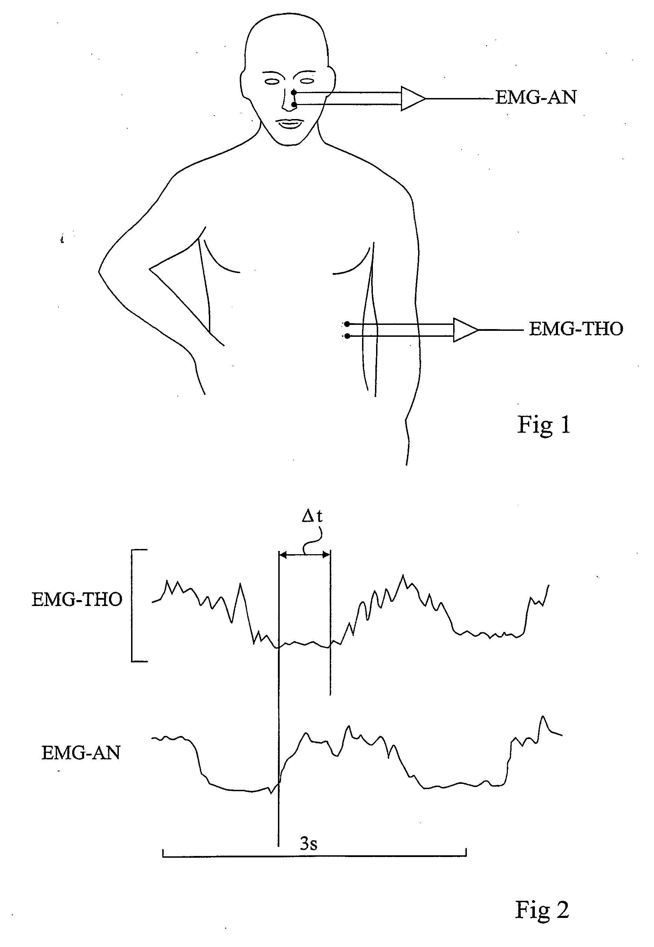

En figure 1, le signal d'électromyogramme au niveau des ailes du nez est appelé EMG-AN, et le signal d'électromyogramme au niveau du thorax est appelé EMG-THO. Le signal d'électromyogramme au niveau du thorax est relevé, par exemple, pour le muscle diaphragmatique pariétal entre les septième et huitième côtes. Il est bien entendu que ces signaux d'électromyogrammes liés à la partie basse du système respiratoire pourront, dans certains cas de paralysie, être relevés à d'autres niveaux. Les électromyogrammes relevés sont indicatifs de l'activation des muscles des ailes du nez et des muscles thoraciques inspiratoires pendant la respiration d'un patient. Selon un aspect de la présente invention, les électromyogrammes sont relevés de façon non invasive par des électrodes cutanées.

Comme l'illustrent les courbes de la figure 2, pour un patient normal, le signal EMG-AN présente une phase de croissance qui correspond à une phase de contraction musculaire qui précède la phase de contraction des muscles thoraciques inspiratoires d'une durée Δt. Cette durée Δt est couramment appelée en médecine retard rostro-caudal puisqu'elle correspond au décalage entre l'activation des voies supérieures (ou avant) et l'activation des muscles inspiratoires thoraciques (ou arrière).

La présente invention prévoit de mesurer ce retard Δt et de fournir une indication en fonction de ce retard. Plus particulièrement, un dispositif selon la présente invention fournira une mesure de ce retard et le comparera à un seuil pour indiquer qu'un patient est dans un état convenable. La valeur dont ce seuil varie est déterminée en fonction des muscles thoraciques explorés. Par exemple, le retard est plus important si la détection est effectuée au niveau du diaphragme pariétal plutôt que des muscles intercostaux inspiratoires. Par exemple, si le patient est sous ventilateur respiratoire, on indiquera que ce ventilateur peut être débranché, quand le retard devient inférieur à une durée de l'ordre de 500 ms. En effet, une période de respiration moyenne est de l'ordre de 5 secondes et on considère qu'un patient est dans un état satisfaisant quand ce retard rostro-caudal est de l'ordre de 0,2 à 0,3 seconde. De façon générale, on pourra utiliser le signal indiquant que le retard rostro-caudal est ou devient inférieur à un seuil, ou au contraire devient supérieur à ce seuil pour fournir un signal de diagnostic. Par exemple, dans le cadre d'opérations d'anesthésie, on veillera à ce que le retard rostro-caudal ne devienne pas supérieur à un seuil déterminé.

Ainsi, la présente invention prévoit un dispositif permettant de relever les électromyogrammes EMG-AN et EMG-THO, de les soumettre à un traitement de façon à éliminer divers signaux parasites, de mesurer de façon claire le retard entre les instants de début de croissance de ces signaux, et de comparer ce retard à un seuil pour donner une indication d'état du patient quand le signal est inférieur au seuil. Dans une application à un respirateur, le signal de comparaison, quand il correspond à une autorisation de débranchement d'un respirateur peut par exemple prendre la forme d'un voyant clignotant disposé sur le dispositif ventilatoire, d'une sonnerie, ou d'un signal fourni à distance sur un écran de contrôle.

Bien entendu, de préférence, le dispositif pourra également comprendre des moyens pour calculer la moyenne du retard sur plusieurs cycles respiratoires, de façon à fournir une moyenne glissante. Le dispositif comprendra alors des moyens pour déterminer que cette moyenne glissante reste supérieure à un seuil pendant une durée déterminée, par exemple une durée de l'ordre de quelques minutes à une heure selon le type de diagnostic recherché.

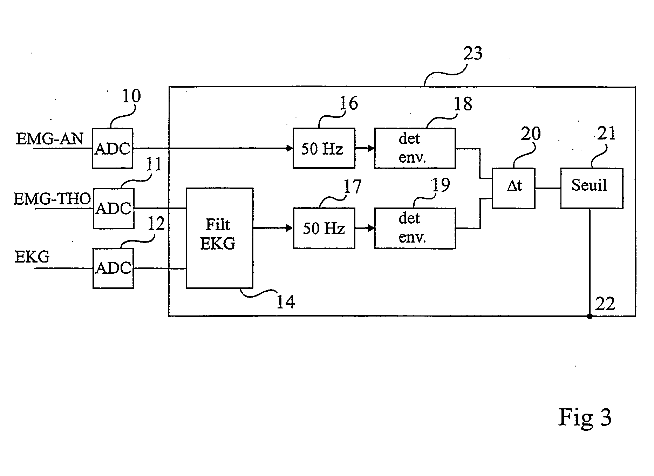

La figure 3 représente un exemple de réalisation d'un dispositif selon la présente invention. Les signaux EMG-AN et EMG-THO, ainsi qu'un signal d'électrocardiogramme EKG sont fournis à des convertisseurs analogique-numérique (ADC) 10, 11 et 12. Les signaux EMG-AN, EMG-THO, et EKG auront de préférence été prétraités, c'est-à-dire par exemple amplifiés et préfiltrés avant conversion analogique-numérique.

Ensuite, les signaux EMG-THO et EKG sont fournis à un premier filtre 14 qui élimine, des signaux d'électromyogramme du diaphragme, les signaux dus aux battements cardiaques. Un filtrage similaire peut être réalisé sur les signaux d'électromyogramme des ailes du nez bien que cela ne soit pas représenté. Ensuite, les signaux sont fournis à des filtres éliminant la fréquence du secteur (couramment le 50 hertz) 16 et 17. Après quoi, des dispositifs 18 et 19 permettent de fournir les enveloppes des signaux EMG-AN et EMG-THO numérisés. Les sorties des détecteurs d'enveloppe 18 et 19 sont fournies à un moyen de calcul 20 du retard Δt entre le début de la montée des signaux d'électromyogramme d'ailes du nez et de diaphragme. Ce retard Δt est fourni à un dispositif 21 de comparaison de la valeur Δt à un seuil. Il peut également être prévu comme on l'a indiqué précédemment, divers dispositifs de détermination d'une moyenne glissante, et de comparaison à un seuil pendant une certaine durée, un signal de validation de sortie 22 étant fourni seulement quand certaines conditions supplémentaires de durée sont remplies.

On a décrit précédemment l'ensemble des éléments 14 à 21 comme des éléments matériels. En fait, en pratique, l'ensemble des fonctions réalisées par ces divers éléments pourra avantageusement être réalisé à l'intérieur d'un processeur numérique, par exemple un micro-ordinateur, recevant les signaux EMG-AN, EMG-THO et EKG numérisés et fournissant le signal de validation 22.

Bien que cela ne soit pas représenté, le dispositif 21 de comparaison à un seuil sera de préférence un dispositif de comparaison à un seuil réglable, ajustable par le personnel infirmier ou médical selon la mesure que l'on cherche à effectuer.

## Revendications

1. Dispositif d'analyse de l'état respiratoire d'un patient comprenant :
des moyens de détermination de l'activité électromyographique (EMG-AN) au niveau des ailes du nez d'un patient et du début des contractions nasales ;
des moyens de détermination de l'activité électromyographique (EMG-THO) au niveau du thorax du patient et du début des contractions inspiratoires thoraciques ;
des moyens de détermination du retard rostro-caudaly (20) entre les débuts des contractions nasales et des contractions inspiratoires thoraciques ;
**caractérisé par** comprenant en outre
des moyens de comparaison dudit retard à un seuil (21) ; et
des moyens de fourniture d'un signal d'alarme quand ledit retard est supérieur audit seuil.

2. Dispositif de contrôle d'un ventilateur respiratoire comprenant :
des moyens de détermination de l'activité électromyographique (EMG-AN) au niveau des ailes du nez d'un patient et du début des contractions nasales ;
des moyens de détermination de l'activité électromyographique (EMG-THO) au niveau du thorax du patient et du début des contractions inspiratoires thoraciques ;
des moyens de détermination du retard rostro-caudale (20) entre les débuts des contractions nasales et des contractions inspiratoires thoraciques ;
**caractérisé par** comprenant en outre des moyens de comparaison dudit retard à un seuil (21) ; et
des moyens de fourniture d'un signal autorisant l'arrêt du ventilateur quand ledit retard est inférieur audit seuil.

3. Dispositif selon la revendication 1 ou 2, dans lequel les moyens de détermination d'activité électromyographique comprennent des électrodes cutanées.

4. Dispositif selon la revendication 3, comprenant en outre :
des moyens de prétraitement des signaux fournis par lesdites électrodes ;
des convertisseurs analogique-numériques (10,11) adaptés à recevoir lesdites signaux après le prétraitement ; et
des moyens numériques de filtrage (16,17) et de mise en forme (18,19) des signaux de sortie des convertisseurs avant les opérations de comparaison.

5. Dispositif selon la revendication 4, comprenant en outre d'autres électrodes cutanées pour capter un signal d'ECG ; et dans lequel les moyens numériques de filtrage (96,17) sont adaptés à faire un filtrage adaptatif de signal cardiaque utilisant ledit signal d'ECG et un filtrage à la fréquence du secteur.

6. Dispositif selon la revendication 4, dans lequel les moyens de prétraitement comprennent des moyens d'amplification et de préfiltrage.

7. Dispositif selon la revendication 1 ou 2, comprenant en outre des moyens de détermination de la moyenne glissante du retard rostro-caudal sur plusieurs phases respiratoires.

8. Dispositif selon la revendication 1 ou 2, dans lequel ledit seuil est réglable entre 200 et 1000 ms.

## Claims

1. A device for analyzing a patient's respiratory state, comprising:
means for determining the electromyographic activity (EMG-AN) at the level of the alae of a patient's nose and the beginning of nasal contractions;
means for determining the electromyographic activity (EMG-THO) at the level of the patient's thorax and the beginning of thoracic inspiratory contractions;
means (20) for determining the rostro-caudal delay between the beginnings of the nasal contractions and of the thoracic inspiratory contractions;
**characterized in that** it further comprises:
means (21) for comparing said delay with a threshold; and
means for generating an alarm signal when said delay is greater than said threshold.

2. A device for controlling a respiratory ventilator comprising:
means for determining the electromyographic activity (EMG-AN) at the level of the alae of a patient's nose and the beginning of nasal contractions;
means for determining the electromyographic activity (EMG-THO) at the level of the patient's thorax and the beginning of thoracic inspiratory contractions;
means (20) for determining the rostro-caudal delay between the beginnings of the nasal contractions and of the thoracic inspiratory contractions;
**characterized in that** it further comprises:
means (21) for comparing said delay with a threshold; and
means for providing a signal authorizing the ventilator to be stopped when said delay is smaller than said threshold.

3. The device of claim 1 or 2, wherein the electromyographic activity determination means comprise skin electrodes.

4. The device of claim 3, further comprising:
preprocessing means of the signals from said electrodes;
analog-to-digital converters (10, 11) adapted to receive said signals after the preprocessing; and
digital filtering (16, 17) and shaping means (18, 19) of the output signals of the converters before the comparison operations.

5. The device of claim 4, further comprising the skin electrodes for sensing an ECG signal, and wherein the digital filtering means (16, 17) are adapted for implementing an adaptive heart signal filtering using said ECG signal and a filtering at the mains frequency.

6. The device of claim 4, wherein the preprocessing is performed by amplification and prefiltering means.

7. The device of claim 1 or 2, further comprising means for determining the sliding average of the rostro-caudal delay over several respiratory phases.

8. The device of claim 1 or 2, wherein said threshold is settable between 200 and 1,000 ms.

## Patentansprüche

1. Vorrichtung zum Analysieren des Atmungszustands eines Patienten, welche Folgendes aufweist:
Mittel zum Bestimmen der elektromyographischen Aktivität in Höhe der Nasenflügel eines Patienten (EMG-AN) und des Beginns nasaler Kontraktionen;
Mittel zum Bestimmen der elektromyographischen Aktivität (EMG-THO) in Höhe des Thorax bzw. Brustkorbes des Patienten und den Beginn inspiratorischer thorakaler Kontraktionen;
Mittel (20) zum Bestimmen der rostro-caudalen Verzögerung zwischen den Anfängen der nasalen Kontraktionen und der inspiratorischen thorakalen Kontraktionen;
**dadurch gekennzeichnet, dass** sie ferner Folgendes aufweist:
Mittel (21) zum Vergleichen der Verzögerung mit einem Grenzwert; und
Mittel zum Erzeugen eines Alarmsignals, wenn die Verzögerung größer als der Schwellenwert ist.

2. Vorrichtung zum Steuern eines Beatmungsgerätes, welche Folgendes aufweist:
Mittel zum Bestimmen der elektromyographischen Aktivität (EMG-AN) in der Höhe der Nasenflügel der Nase eines Patienten und des Beginns nasaler Kontraktionen;
Mittel zum Bestimmen der elektromyographischen Aktivität (EMG-THO) in Höhe des Thorax bzw. Brustkorbes des Patienten und des Beginns inspiratorischer Kontraktionen des Thorax.
Mittel (20) zum Bestimmen der rostro-caudalen Verzögerung zwischen den Anfängen der nasalen Kontraktionen und der inspiratorischen thorakalen Kontraktionen;
**dadurch gekennzeichnet, dass** sie ferner Folgendes aufweist:
Mittel (21) zum Vergleichen der Verzögerung mit einem Grenzwert; und
Mittel zum Liefern eines Signals, welches das Beatmungsgerät ermächtigt, anzuhalten, wenn die Verzögerung geringer ist als der Schwellenwert.

3. Vorrichtung nach Anspruch 1 oder 2, wobei die Mittel zur Bestimmung der elektromyographischen Aktivität Hautelektroden aufweisen.

4. Vorrichtung nach Anspruch 3, welche ferner Folgendes aufweist:
Vorverarbeitungsmittel für die Signale von den Elektroden;
Analog-zu-Digital-Wandler (10, 11), die angepasst sind, um die Signale nach der Vorverarbeitung zu empfangen; und
digitale Filter- (16, 17)- und Formmittel (18, 19) für die Ausgangssignale der Wandler vor den Vergleichsoperationen.

5. Vorrichtung nach Anspruch 4, welche ferner Hautelektroden zum Abfühlen eines EKG-Signals aufweist, und wobei die digitalen Filtermittel (16, 17) geeignet sind, zur Implementierung der adaptiven Filterung des Herzsignals unter Verwendung des EKG-Signals und zu einer Filterung bei der Netzfrequenz.

6. Vorrichtung nach Anspruch 4, wobei die Vorverarbeitung durch Verstärkungs- und Vorfilterungsmittel durchgeführt wird.

7. Vorrichtung nach Anspruch 1 oder 2, welche ferner Mittel zum Bestimmen des gleitenden Mittelwerts der rostro-caudalen Verzögerung über mehrere Atmungsphasen hinweg aufweist.

8. Vorrichtung nach Anspruch 1 oder 2, wobei der Schwellenwert auf zwischen 200 und 1000 ms einstellbar ist.
